(19) Europäisches Patentamt
European Patent Office
Office européen des brevets

(11) **EP 4 372 681 A2**

(12) **EUROPEAN PATENT APPLICATION**

(43) Date of publication:
**22.05.2024 Bulletin 2024/21**

(21) Application number: **23210474.5**

(22) Date of filing: **16.11.2023**

(51) International Patent Classification (IPC):
**G06T 7/00** *(2017.01)*  **G06T 7/50** *(2017.01)*

(52) Cooperative Patent Classification (CPC):
**G06T 7/50; G06T 7/0012;** A61B 5/4547; A61C 7/00;
G06T 2207/10016; G06T 2207/20081;
G06T 2207/20084; G06T 2207/30036

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.11.2022  US 202263426088 P
17.11.2022  US 202263426084 P
17.11.2022  US 202263426078 P**

(71) Applicant: **Dror Orthodesign Limited
9546103 Jerusalem (IL)**

(72) Inventors:
• **AVNI, Yossi
Caesarea (IL)**
• **HADDAD, Eliyahu
Har Adar (IL)**
• **SHVETS, Moshe
Beit Meir (IL)**
• **SUCHARD, Eitan
Chicago, 60087 (US)**

(74) Representative: **Kancelaria Eupatent.pl Sp. z.o.o
Ul. Kilinskiego 185
90-348 Lodz (PL)**

(54) **MISALIGNMENT CLASSIFICATION, DETECTION OF TEETH OCCLUSIONS AND GAPS, AND GENERATING FINAL STATE TEETH ALIGNER STRUCTURE FILE**

(57)    A method including: obtaining a plurality of images in which front teeth of a subject are visible; performing segmentation on selected images from the plurality of images to create a first segmentation mask and labeling each tooth in the selected images to provide a detailed segmentation map; generating a depth map of the front teeth; calculating a horizontal gradient of the depth map and a vertical moving average of a plurality of pixels of the horizontal gradient to receive depth gradients and flagging depth gradients where the vertical moving average exceeds a predefined threshold or is classified by an Artificial Neural Network or other machine learning model as abnormal; inputting the depth gradients and detailed segmentation map into a classifier to determine whether the front teeth are within predetermined parameters; and receiving a go or no-go classification from the classifier.

FIG. 1

100 → 102 Acquire set of 2D images
Filter for noise – 102.1
Extract mouth rectangle – 102.2

104 Perform segmentation on 2D images.

104.1 Preprocess images to segment teeth from other.

104.2 Use segmentation to label each tooth.

106 Generate a points cloud depth map of the front teeth

108 Scanning and smoothing depth map to receive depth gradients and flag abnormal gradients

110 Input depth gradients into a classifier

112 Receive a go or no-go classification from the classifier

EP 4 372 681 A2

**Description**

**This** patent application claims the benefit of U. S. Provisional Patent Application No. 63/426,078, filed November 17, 2022, U. S. Provisional Patent Application No. 63/426,084, filed November 17, 2022, U. S. Provisional Patent Application No. 63/426,088, filed November 17, 2022, which are incorporated in their entirety as if fully set forth herein.

FIELD OF THE INVENTION

The field of the invention is the use of advanced physical dental treatment with the aid of front teeth state image analysis at the teeth alignment diagnosis process with regards to specific pre-defined dental/orthodontic teeth conditions and disorders such as Class I, Class II, and Class III states. The field of the invention includes teeth alignment devices that are guided by preliminary image analysis. The field of the invention includes teeth segmentation in 2D and 3D, data acquisition of 3D teeth models, and their gradual update for improved accuracy using several teeth images.

BACKGROUND OF THE INVENTION

Accurate depth map algorithms require prior knowledge of pin-hole parameters of the camera which consist, at the very least, of an intrinsic and of an extrinsic matrix with some cameras also requiring 6 distortion parameters as well. Yet, the human visual cortex of a dentist does not directly compute any such matrices and still a dentist is able to assess whether front teeth, including the canine teeth, overlap or have gaps between them from a front view. This fact motivates the presented algorithm.

State-of-the-art neural network algorithms, such as IDR from Lior Yariv et. al., rely on modeling zero sets of space points but are very slow in the scale of at least 30 minutes, require an accurate masking of almost continuous surfaces and are not suitable for multiple surfaces such as in the case of teeth. In addition, such algorithms require chessboard calibrations which requires a printed chessboard and calculation of a two dimensions 3x3 intrinsic matrix with two focal points and two offsets one for horizontal and one for vertical coordinates:

$$\begin{pmatrix} f_x & 0 & c_x \\ 0 & f_y & c_y \\ 0 & 0 & 1 \end{pmatrix} \tag{1}$$

And the extrinsic matrix consists of a three-dimensional rotation matrix R and a homogenous translation vector $(t_x, t_y, t_z, 1)$ both represented in the same 4x4 matrix in homogenous coordinates:

$$\begin{pmatrix} & & & t_x \\ & R & & t_y \\ & & & t_z \\ 0 & 0 & 0 & 1 \end{pmatrix} \tag{2}$$

The calibration is cumbersome and although the intrinsic matrix does not change from image to image, the extrinsic matrix does, and it requires a chessboard calibration as done, for example, in open cv by using:

cv.findChessboardCorners
cv.cornerSubPix

$$\text{OK, matrix, distortion\_vector, rvecs, tvecs } = \text{cv.calibrateCamera} \tag{3}$$

$$R = \text{cv.Rodrigues(rvecs)}[0]$$

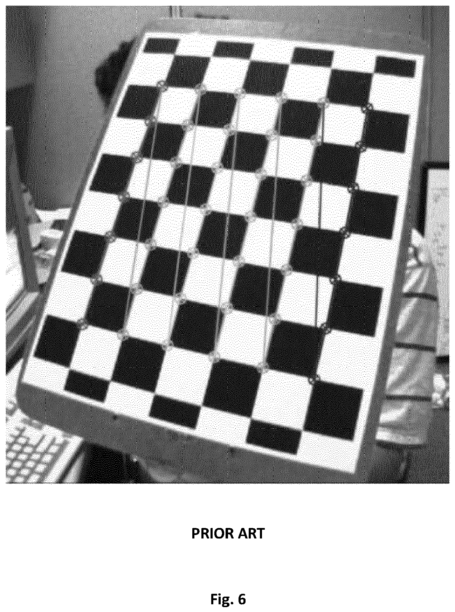

Figure 6 illustrates an image of a chessboard used for a prior art calibration. For example, the chessboard is used in the calculation of the intrinsic and extrinsic camera matrices. In Fig. 6, the normal to the chessboard must be with the same direction of normal to middle point of the observed surface (applicable, for example, to front teeth), otherwise the angles of the chessboard do not reflect the extrinsic matrix of observed surface.

Another option is to use vertical summation of Gaussian filter of horizontal gradients of a depth map. Although the pin hole camera model is required for an accurate depth assessment, identification of exact depth is not required in order to identify large horizontal gradients in the depth map.

High gradients of this type can be detected by using a rescaled MIDAS 384 x 384 monocular depth map from a single image. High gradients at the edges of the image are ignored because they are the result of rounding dental arch. The algorithm also filters out naturally occurring horizontal gradients of the depth map or due to teeth curvature. The problem in using a monocular depth map is that its errors are too high for commercial purposes.

Other algorithms such as Meshroom™, which uses AliceVision®, match key points across different images, based on key point matching through the SURF or SIFT algorithms. Then from these key points, by using the RANSAC (Random Sample Consensus) algorithm, a partial subset is used for comparison and depth calculation. If n key points are in one image and m key points are in another, n * m comparisons are performed. The result is not dense and of low quality, which performs worse than the monocular MIDAS algorithm. There are better depth map algorithms such as LSD-SLAM that optimize a Lie Group transformation by using its derivatives as Lie Algebra member along with the Newton-Gauss optimization algorithm. These algorithms are semi-dense and are better than feature based depth map algorithms, however, they are not fully parallel.

## SUMMARY OF THE INVENTION

According to the present invention there is provided a method including: obtaining a plurality of images in which front teeth of a subject are visible; performing segmentation on selected images from the plurality of images to create a first segmentation mask and labeling each tooth in the selected images to provide a detailed segmentation map; generating a depth map of the front teeth; calculating a horizontal gradient of the depth map and a vertical moving average of a plurality of pixels of the horizontal gradient to receive depth gradients and flagging depth gradients where the vertical moving average exceeds a predefined threshold or is classified by an Artificial Neural Network or other machine learning model as abnormal; inputting the depth gradients and detailed segmentation map into a classifier to determine whether the front teeth are within predetermined parameters; and receiving a go or no-go classification from the classifier.

According to further features performing segmentation includes a preprocessing step of denoising and features space analysis adapted to segment teeth from other elements in each of the selected images.

According to still further features in the described preferred embodiments the detailed segmentation map is generated by employing an artificial neural network (ANN) or other trained machine learning model to recognize each tooth and label each tooth with clear edges thereof.

According to still further features the depth map is generated by a specialized U-Net trained on a dataset of images of teeth in different configurations. According to still further features the classifier is a convolution neural network (CNN).

According to another embodiment there is provided a method for generating a depth map from a 2-dimensional image, including: training a depth map U-Net neural network on a dataset of images, wherein a depth value of each pixel in each image is known; inputting the 2-dimensional image to the depth map U-Net; and outputting, by the depth map U-Net, the depth map of the 2-dimensional image.

According to further features the depth map U-Net has a three-channel encoder and a two-channel decoder. According to still further features the three-channel encoder has a left propagation path, a right propagation path and a middle propagation path. According to still further features the middle propagation path is self-supervised.

According to another embodiment there is provided a non-transitory computer-readable medium includes instructions stored thereon, that when executed on a processor perform a method of generating a final state teeth aligner structure file, including: receiving a 3-dimensional (3D) scan of a dental arch; analyzing the 3D scan to get a manifold of teeth representing a final aligned teeth position in a 3D space; converting the manifold of teeth into a points cloud; generating a representation of a mold by expanding the manifold of teeth along surface normal vectors thereof; combining a points cloud of a balloon structure to the points cloud of the manifold to receive an aligner points cloud; converting the aligner points cloud into a representation of an aligner in a 3D printable file format.

According to further features the method further includes printing an aligner on a 3D printer from the representation of the aligner in the 3D printable file format.

## BRIEF DESCRIPTION OF THE DRAWINGS

Various embodiments are herein described, by way of example only, with reference to the accompanying drawings, wherein:

  FIG. 1 is a flow diagram of a software solution go/no-go diagnosis process 100;
  FIGS. 2a-c are stages in the 2D segmentation process;

FIGS. 3a-e are various components used to create a dataset for training the U-Net;

FIG. 4a-c are screenshots from an example horizontal scan of the system;

FIG. 5a-c are screenshots from an example vertical scan of the system;

FIG. 6 is an image of a chessboard used for a prior art calibration;

FIG. 7A is a diagram of an example encoder of the depth map U-Net of the invention;

FIG. 7B is diagram of a regular autoencoder;

FIG 8A is a depth map encoder when the transformation between the left and right image is large;

FIG 8B is a depth map decoder when the transformation between the left and right image is large;

FIGS. 9A-E are STL and points cloud images of a dental arch from a 3D scan;

FIG. 10 is a diagrammatic representation of a cross-sectional view of an aligner seated over a tooth.

DESCRIPTION OF THE PREFERRED EMBODIMENTS

The present invention is related to a system that includes a device (a special mouthpiece with an inflated balloon and mobile pump) and a method based on AI models from diagnostics (Go/No-Go test software), treatment plans analysis (software), and execution (using the special device) to align teeth without braces. The present invention is specifically directed to the Go/No-Go test software aspect of the system.

There is presently described a method and system for providing a diagnostic tool that is able to analyze images (e.g., from a video stream) of a subject's mouth and decide whether the teeth are misaligned (usually falling into the category of Class I or Class II) within predefined parameters that can be treated by a given proprietary device and process (a mouthpiece with inflatable balloon). The methods and systems described hereafter are adapted to be performed by one or more systems that include, inter-alia, a computer, memory, and processor. A non-transitory computer-readable medium includes instructions stored thereon, that when executed on the processor perform one or more of the tasks detailed hereafter.

The images capture at least the 12 front teeth, six on the top and six on the bottom. Even though there are various numbering and labeling systems for numbering / labeling the teeth, a simple labeling system is used herein to identify the relevant teeth. The top front teeth (in the upper / maxillary arch) are the right central/1st incisor, the right lateral/2nd incisor, the right cuspid / canine, the left central/1st incisor, the left lateral/2nd incisor, and the left cuspid / canine (universal numbers 8, 7, 6, 9, 10 and 11 respectively). The bottom front teeth (in the lower / mandibular arch) are the right central/1st incisor, the right lateral/2nd incisor, the right cuspid / canine, the left central/1st incisor, the left lateral/2nd incisor, and the left cuspid / canine (universal numbers 25, 26, 27, 24, 23 and 22 respectively).

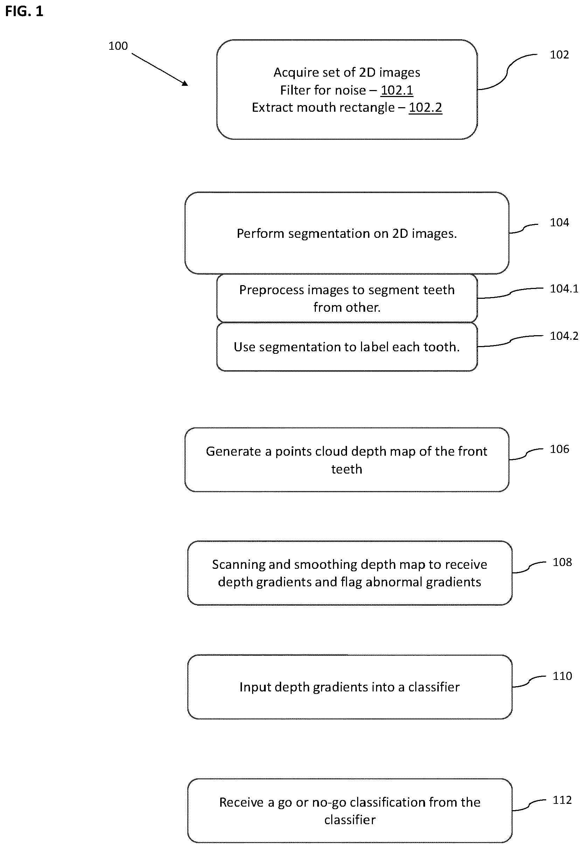

Referring now to the drawings, Figure 1 illustrates a flow diagram of a software solution go/no-go diagnosis process 100. The process starts at Step 102 which entails acquiring a set of images (sequence of images) of front teeth of a subject, taken from several different front views / angles.

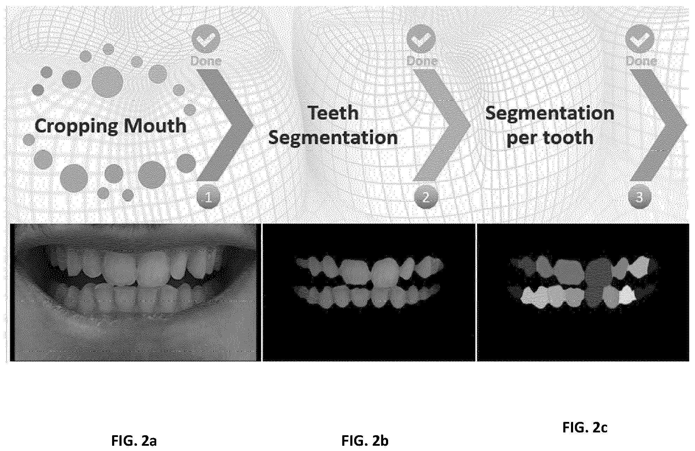

In Step 104 the system performs 2D segmentation. This step includes a number of sub-steps as follows. First, the images are cropped so that only the mouth is visible (Fig. 2a). Next, the teeth are segmented from the mouth (Fig. 2b). Finally, the individual teeth are segmented and labeled, e.g., with a label or color (Fig. 2c).

In order to perform the 2D segmentation, the system invokes a sub-process of "down-sampling" the images which includes the segmentation of teeth, gums, and lips. In example embodiments, U-Net technology, which is a neural network commonly used in medical image segmentation, is used for the down-sampling / segmentation sub-process. The U-Net has a conventional structure of an encoder (which is, for e.g., a convolutional neural network-CNN) and a decoder, with conventional skip-connections between layers of the encoder and layers of the decoder.

The U-Net achieves a two-dimensional segmentation which is mapped onto a three-dimensional mesh. In a preferred embodiment, two different segmentation neural networks were used. The first was FCN_RESNET50 and the second was a proprietary U-Net.

The proprietary U-Net receives a down-sampled RGB image of dimensions 448 * 448 * 3 and outputs a 448 * 448 * 36 image. The output channels are Hot-Ones which is a term well known in Machine Learning and Artificial Neural Networks. Humans have 9 types of teeth, 8 without Wisdom teeth. In total there are 32 adult teeth. The described U-Net model is trained on 12 front teeth (upper six teeth and lower six teeth) in which each type (central incisor, lateral incisor, cuspid) repeats 4 times, top-right, top-left, bottom-right, and bottom-left. In a more general case that covers all mouth teeth (as opposed to the instant example embodiment that only covers the 12 front teeth), the example embodiment has 36 channels. 34 channels are for the teeth, including Wisdom teeth and two channels are for gums and lips. If a pixel in the input image belongs to a bottom-right main molar tooth, the channel with index 1 should output 1 and the remaining 35 channels should output 0.

The training of the U-Net of the preferred embodiment is not conventional. The invention trains the encoder of the U-Net separately from the segmentation task. The encoder of the U-Net is also a component of a separate Auto-encoder that is used solely for the purpose of training the encoder. The auto-encoder is trained to receive a 448 * 448 * 3 input which is encoded into a 7 * 7 * 1024 - dimensional vector. The compression ratio is (448 * 448 * 3) / (7 * 7 * 1024) = 12. Then a decoder up-samples the output of the encoder, 7 * 7 * 1024 back into 448 * 448 * 3.

The combination of down-sampling by an encoder and up-sampling by a decoder is a well-known technology in the prior art which is called auto-encoder. Auto-encoders create an information bottleneck / bridge by down-sampling the image. The advantage of auto-encoders is that they are noise filters as they learn to represent only real features of an image but not the noise component of the image. The autoencoder is trained to output an image as close as possible to the original image by reducing the sum of square errors between the original image and the output image, pixel by pixel.

Sections 1-4 can be broken down into more detailed steps.

Recalling these 4 general steps:

1. The Go/No-Go module receives a stream of front teeth images, frame by frame as input data (Step 102).

2. Each image frame is pre-processed for noise reduction (denoising) and features space analysis, to segment teeth from other elements in each of the selected images (Step 104.1).

3. Each image frame is semantically segmented by a customized 2D U-Net ANN (Artificial Neural Network). Each tooth is recognized, receives a different color, or label with clear edges (Step 104.2).

4. Each image frame is a lizard for the 3D points cloud and in particular to points with significant meaning like gaps between the front teeth and steep depth gradients due to overlapping. This way, the 2D semantic segmented objects (as defined in the labeling process) become 3D segmentation without the explicit need for voxel-based 3D U-Nets. This is a unique and innovative process that reduces data augmentation and process complexity.

Breaking the previous sections 1-4 down we have the following more detailed description.

The algorithm starts with data acquisition through a smartphone camera as a video.

1) The video is analyzed in rate of at least 6 frames per second, FPS, out of the usual 30 FPS and if necessary, images are filtered for noise (Step 102.1).

2) The algorithm uses face pose points to extract the mouth rectangle for each selected frame (Fig. 2a). It also calculates which face poses are within -30 to +30 degrees rotation in the XZ plane where X is the horizontal value and Z is the depth (Step 102.2).

3) First segmentation mask is generated which is 1 for a tooth and 0 elsewhere (Step 104.1). Figure 2b shows the result of the first segmentation mask. The artificial neural network (or other machine language model) is trained on a very large set of images to recognize and distinguish teeth from lips and gums.

4) A detailed segmentation is done for 12 tooth types, lips, gums, gaps between teeth, non-front teeth as one single class, and others unknown objects as one single class. Altogether there are 17 classes. Figure 2c shows the segmentation where each of the 12 tooth types are labeled by a different color and the rest of the classes are depicted in black (or no color). In some embodiments, the unknown objects are unified with the class of gaps between teeth, as the segmentation performance is better with this tweak of the algorithm (Step 104.2).

5) In step 106 a depth map is calculated on the entire face, using depth clues from the entire face. This is done in one of 3 methods,

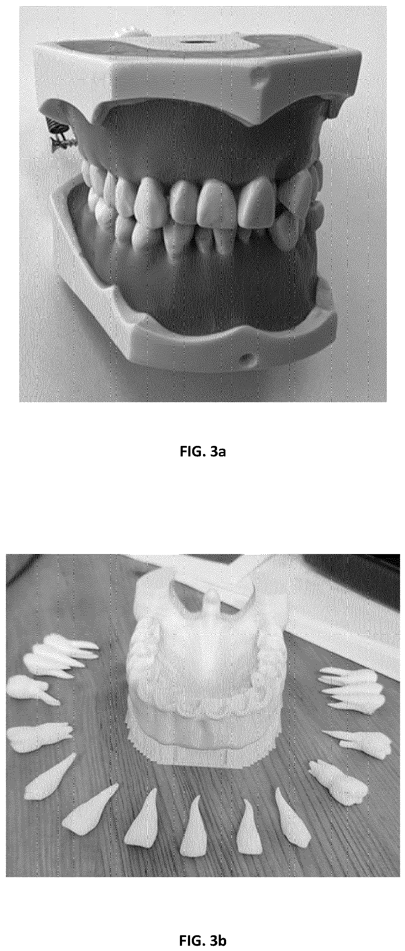

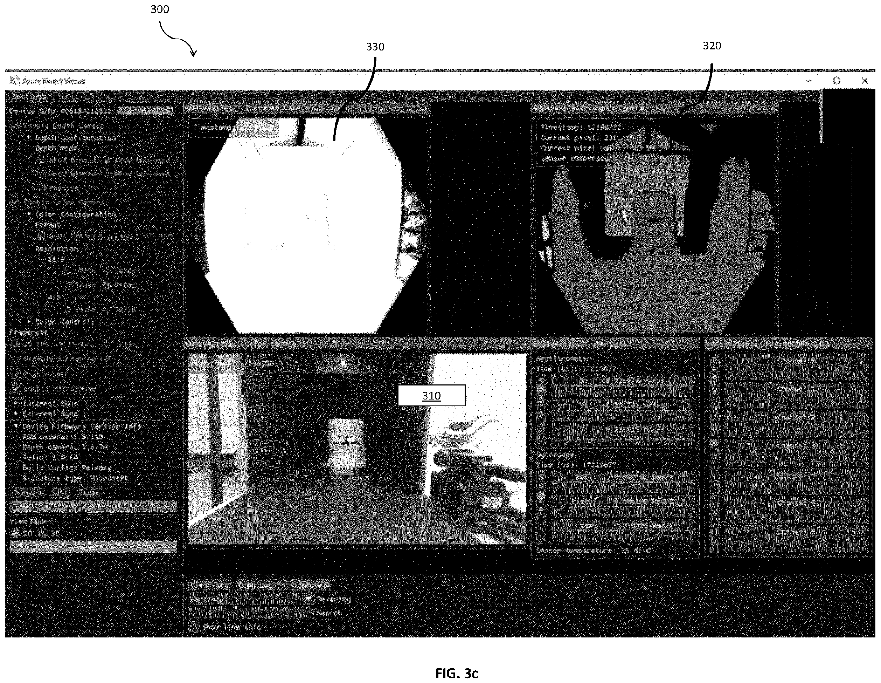

5.1. Proprietary/specialized U-Net that receives at least two images, one from the Left and one from the Right. The specialized U-Net was trained on thousands of images of a model set of teeth where in each image the teeth are modeled in a different configuration. Figures 3a-e depict various components used to create a dataset for training the U-Net. Figure 3a depicts a physical model of a jaw according to one example configuration. Figure 3b depicts the jaw of the model with the movable teeth removed. The teeth are arranged in different positions and at different angles in the model jaw. Figure 3c depicts a graphical user interface (GUI) 300 including images from a color camera 310, a depth camera 320 and an infrared camera 330.

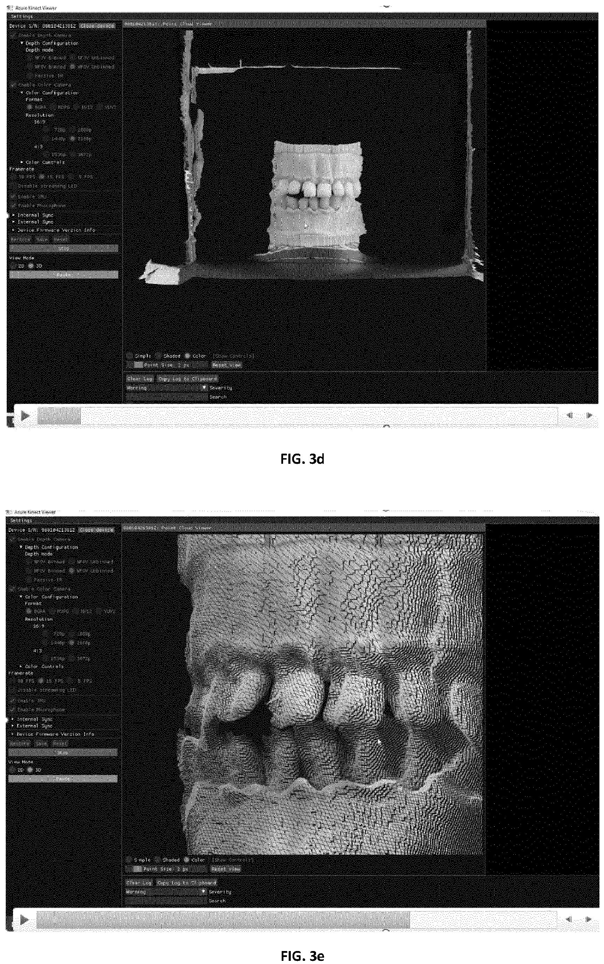

Figure 3d is a zoomed-out view of a point cloud viewer of the GUI. Figure 3e is a zoomed-in view of the same image in the point cloud viewer of the GUI.

5.2. LSD-SLAM. Large-Scale Direct Simultaneous Localization and Mapping algorithm is a semi dense algorithm.

5.3. Feature based method. This latter option was tried with Alice Vision; however, the results were not satisfactory. The invention does not rule out the possibility that other feature-based depth map algorithms can work well.

6) The depth map is cropped by the mouth rectangle and by the first segmentation mask.

7) The first segmentation mask (1 on teeth, 0 elsewhere) is cropped in the mouth rectangle.

8) The detailed segmentation is masked using the first segmentation mask and is cropped by the mouth rectangle to provide a detailed segmentation map.

9) In step 108 the depth map horizontal gradients are calculated. Then the horizontal gradients are used to calculate a vertical moving average of 11 values along 11 vertical pixels. This paradigm works well also with 13 vertical pixels.

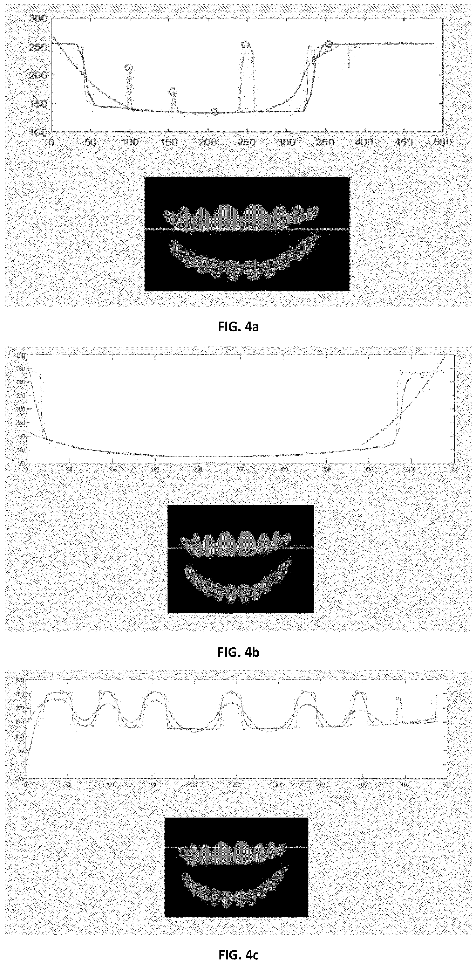

A threshold for interesting moving average of these is used to flag regions of interest. Figures 4a, 4b and 4c depict screenshots from an example horizontal scan of the system.

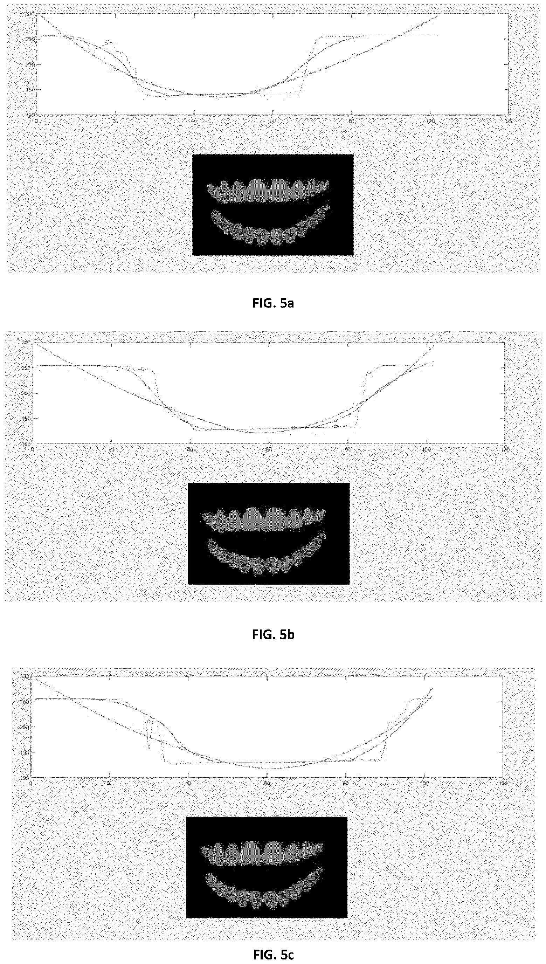

10) The moving average of vertical sums of horizontal depth map gradients is an input to a Go/No-Go classification convolutional neural network along with the detailed segmentation map. The classification neural network outputs a Go/No-Go decision per each tooth separately and to the entire process. Figures 5a-c depict screenshots from an example vertical scan of the system.

Both the teeth alignment and the Go/No-Go algorithm are based on front teeth segmentation as described in (8) e.g., by FCN_RESNET50 which attaches a number to each tooth type according to the following list:

The segmentation of front teeth is as follows:

Others: 0, Lips: -1, Gums: -2, Gaps between teeth: -3, All other teeth: -4, Upper left canine: 11, Upper left 2nd incisor: 10, Upper left 1st incisor: 9, Upper right 1st incisor: 8, Upper right 2nd incisor: 7, Upper right canine: 6, Lower right canine: 27, Lower right 2nd incisor: 26, Lower right 1st incisor: 25, Lower left 1st incisor: 24, Lower left 2nd incisor: 23, and Lower left canine: 22.

In addition, a depth map is calculated using a proprietary U-Net, LSD-SLAM or a feature-based depth map.

Then the horizontal gradient of the depth map $\dfrac{\partial Depth(y,x)}{\partial x}$ is calculated and a vertical moving average of 11 pixels of this gradient $G(y = i, x) = \dfrac{1}{11}\sum_{j=i}^{j=i+10} \dfrac{\partial Depth(j,x)}{\partial x}$. Where this average exceeds a predefined threshold, a depth gradient flag is set to 1. Alternatively the depth gradients that are classified by an Artificial Neural Network (ANN) (or other trained machine learning model) as abnormal are flagged.

In step 110, The depth gradients $G(y, x)$ are the input of a classifier, for e.g., a Go/No-go Convolutional Neural Network or other trained machine learning model. In embodiments, the detailed segmentation map also serves as input for the classifier.

In Step 112, a go or no-go classification is received from the classifier.

Example Embodiment - Detailed Implementation

## The encoder

In the preferred embodiment, the autoencoder was further trained in a rather unusual way. The teeth image size is reduced proportionally by the pre-defined encoder which is a convolutional neural network model using convolutions process and max-pooling as described in the following flow Top-Down Right side of the U-Net network architecture, in this example using expended receptive field (input layer). In the following description, for the sake of simplicity, a batch-norm or instance-norm layer after each convolutional layer is omitted in the following description:

$$x: 448 * 448 * 3 \rightarrow C_1 \rightarrow$$

$$448 * 448 * 32 \rightarrow S_1 \rightarrow$$

$$x_1: 224 * 224 * 32 \rightarrow C_2 \rightarrow$$

$$224 * 224 * 64 \rightarrow S_2 \rightarrow$$

$$x_2: 112 * 112 * 64 \rightarrow C_3 \rightarrow$$

$$112 * 112 * 128 \rightarrow S_3 \rightarrow$$

$$x_3: 56 * 56 * 128 \rightarrow C_4 \rightarrow$$

$$56 * 56 * 256 \rightarrow S_4 \rightarrow$$

$$x_4: 28 * 28 * 256 \rightarrow C_5 \rightarrow$$

$$28 * 28 * 512 \rightarrow S_5 \rightarrow$$

$$x_5: 14 * 14 * 512 \rightarrow C_6 \rightarrow$$

$$14 * 14 * 1024 \rightarrow S_6 \rightarrow$$

$$V: 7 * 7 * 1024$$

Flow

The variable x denotes the original image where the number 3 denotes the 3 RGB channels. V denotes the output of the decoder.

The variables $x_1$, $x_2$, $x_3$, $x_4$, $x_5$ denote intermediate down sampled image with an increasing number of channels. Each convolutional layer $C_1$, $C_2$, $C_3$, $C_4$, $C_5$, $C_6$ has padding of 1 pixel to each side of the x axis and 1 padding pixel to each side of the y axis. The receptive field is 3 *3 in size. The padding adds 2 to the length of each dimension and the kernel reduces each dimension by 2. The result is that the convolution layers do change the horizontal and the vertical resolutions of the image, however, the number of features is doubled after each convolution or series of convolutions. The invention is not limited to $C_i$ as a single convolution layer. $C_i$ can denote several convolution layers but usually not more than three.

The auto-encoder decoder that is used for the training process is described below. In the following description, for the sake of simplicity, a batch-norm or instance-norm layer after each convolutional layer is omitted in the following description:

$$7 * 7 * 1024 \rightarrow T_6 \rightarrow$$

$$14 * 14 * 1024 \rightarrow \tilde{C}_6 \rightarrow$$

$$z_5 : 14 * 14 * 512 \rightarrow T_5 \rightarrow$$

$$28 * 28 * 512 \rightarrow \tilde{C}_5 \rightarrow$$

$$z_4 : 28 * 28 * 256 \rightarrow T_4 \rightarrow$$

$$56 * 56 * 256 \rightarrow \tilde{C}_4 \rightarrow$$

$$z_3 : 56 * 56 * 128 \rightarrow T_3 \rightarrow$$

$$112 * 112 * 128 \rightarrow \tilde{C}_3 \rightarrow$$

$$\Downarrow \begin{smallmatrix} O \\ W \end{smallmatrix}$$

$$z_2 : 112 * 112 * 64 \rightarrow T_2 \rightarrow$$

$$224 * 224 * 64 \rightarrow \tilde{C}_2 \rightarrow$$

$$z_1 : 224 * 224 * 32 \rightarrow T_1 \rightarrow$$

$$448 * 448 * 32 \rightarrow \tilde{C}_1 \rightarrow$$

$$z : 448 * 448 * 3$$

Where the T layers $T_1$, $T_2$, $T_3$, $T_4$, $T_5$, $T_6$ are Transposed Convolution layers with stride (2,2) and kernel (2,2). The T layers double the dimension but do not change the number of channels / features. The convolution layers $\tilde{C}_1$, $\tilde{C}_2$, $\tilde{C}_3$, $\tilde{C}_4$, $\tilde{C}_5$, $\tilde{C}_6$ have the same structure of the convolution layers of the encoder, which means they have padding 1 to each of the right and left sides of the x axis, padding 1 to each of the sides of the y axis to each of the bottom side and the top side. The padding adds 2 to the length of each dimension. The kernel is 3*3 dimensional and reduces each dimension by 2. Therefore, the convolution layers of the decoder, do not change the x*y resolution, however they halve the number of channels / features except for the last convolutional layer $C_1$.

The 7 * 7 * 1024 output of the encoder V becomes the input of the decoder which tries to reconstruct the image through the output z.

Following is the loss function of the autoencoder:

$$Loss = 0.6 * \|z_5 - x_5\|^2 + 0.3 * \|z_4 - x_4\|^2 + 0.15 * \|z_3 - x_3\|^2 + 0.075$$

$$* \|z_2 - x_2\|^2 + 0.0375 * \|z_1 - x_1\|^2 + 1 * \|z - x\|^2$$

The weight is the dimension of the original image divided by 2 * number of intermediate vectors * dimension of the reduced image. For example, for $\|z_1 - x_1\|^2$ the weight in the loss function is

$$0.0375 = \frac{448 * 448 * 3}{224 * 224 * 32} * \frac{1}{5} * \frac{1}{2}$$

The more values there are, in this case 224 * 224 * 32, the less weight each pixel gets in the loss calculation. The factor 5 is because we want to equally weigh in 5 intermediate vectors and the factor 2 is because we want all these loss functions together to have half the importance of $\|z - x\|^2$.

$\|z - x\|^2$ alone, defines the objective loss to be minimized so why does the algorithm use the other loss functions? The reason is that a combined loss function takes into account that the intermediate vectors will make sense if we want to reconstruct the image in a different neural network, based also on the intermediate vectors of the encoder. We want these intermediate vectors to be useful in Transfer Learning manner, once the trained encoder is used by a separate U-Net and the intermediate vectors are then used for the Skip Connections. In this sense, this method of training an autoencoder with a weighted loss function per intermediate encoder layers in order to later use such outputs in a U-Net, based on Transfer Learning is an inventive step in its own. For this reason, the U-Net and autoencoder method was described at length. Why is it important? U-Nets do not converge easily and there is not much leeway in the choice of the U-Net loss function that can converge, which is usually the Dice Loss. This is especially true when the training set is under several thousand images. Therefore, any boosting method that helps the convergence of U-Nets, even when the training samples are within several thousand labeled images, is very important.

**An example segmentation U-Net**

We have already seen the encoder part of the U-Net which is trained in a separate neural network, namely an encoder-decoder or autoencoder neural network. Once the training is completed, the neural weights of the encoder are not allowed to change anymore. The U-Net training is then focused only on a new decoder.

The U-Net decoder is made of up-sampling bilinear interpolation layers $UP_6$, $UP_5$, $UP_4$, $UP_3$, $UP_2$, and $UP_1$, and convolution layers that do not change the image resolution but reduce the number of channels, $\bar{\bar{C}}_6, \bar{\bar{C}}_5, \bar{\bar{C}}_4, \bar{\bar{C}}_3, \bar{\bar{C}}_2, \bar{\bar{C}}_1$ and concatenation of the output of layers of the encoder $x_5$, $x_4$, $x_3$, $x_2$, $x_1$ with the outputs of the up-sampling, $Cat(x_5; 14 * 14 * 512)$, $Cat(x_4; 28 * 28 * 256)$, $Cat(x_3; 56 * 56 * 128)$, $Cat(x_2; 112 * 112 * 64)$, $Cat(x_1; 224 * 224 * 32)$. Following is the cascade of layers of the U-Net decoder:

$$V: 7 * 7 * 1024 \rightarrow UP_6 \rightarrow$$

$$14 * 14 * 1024 \rightarrow \bar{\bar{C}}_6 \rightarrow$$

$$14 * 14 * 512 \rightarrow Cat(x_5; 14 * 14 * 512) \rightarrow$$

$$14 * 14 * 1024 \rightarrow UP_5 \rightarrow$$

$$28 * 28 * 1024 \rightarrow \bar{\bar{C}}_5 \rightarrow$$

$$28 * 28 * 256 \rightarrow Cat(x_4; 28 * 28 * 256) \rightarrow$$

$$28 * 28 * 512 \rightarrow UP_4 \rightarrow$$

$$56 * 56 * 512 \rightarrow \bar{\bar{C}}_4 \rightarrow$$

$$56 * 56 * 128 \rightarrow Cat(x_3; 56 * 56 * 128) \rightarrow$$

$$56 * 56 * 256 \rightarrow UP_3 \rightarrow$$

$$112 * 112 * 256 \rightarrow \bar{\bar{C}}_3 \rightarrow$$

$$112 * 112 * 64 \rightarrow Cat(x_2; 112 * 112 * 64) \rightarrow$$

$$112 * 112 * 128 \rightarrow UP_2 \rightarrow$$

$$224 * 224 * 128 \rightarrow \bar{\bar{C}}_2 \rightarrow$$

$$224 * 224 * 32 \rightarrow Cat(x_1; 224 * 224 * 32) \rightarrow$$

$$224 * 224 * 64 \rightarrow UP_1 \rightarrow$$

$$448 * 448 * 64 \rightarrow \bar{\bar{C}}_1 \rightarrow$$

$$448 * 448 * 36$$

Alternatively, each of the convolution layers, $\bar{\bar{C}}_6, \bar{\bar{C}}_5, \bar{\bar{C}}_4, \bar{\bar{C}}_3, \bar{\bar{C}}_2, \bar{\bar{C}}_1$ is replaced by several layers in order to allow better expression of the output by the U-Net decoder.

The outputs of the U-Net marks each pixel with a number that tells if a pixel belongs to a tooth, to gums, to lips or to the background. Output images are acquired when the input is from different angles. These outputs of the U-Net are used for classification as an input of a classifier that outputs 1 if teeth alignment can work and 0 otherwise.

In some example embodiments, the segmentation numbers are then projected on 3D point clouds. The 3D point cloud is based on existing algorithms which use local descriptors such as based on SIFT and SURF. These algorithms usually calculate SIFT or SURF descriptors in points which have a high Laplacian which means high curvature. Then the points are tracked through different angles by comparing descriptors as the angle of view gradually changes.

An alternative to using the dedicated U-Net embodiment is to use semi-dense depth map algorithms such as LSD-SLAM which use Lie Algebras to describe differentiable rotation, scaling and translation transformations between

frame $_k$ and frame $_{k+1}$ and calculate an inverse depth map for each frame in a video.

Self-Supervised Depth Map Algorithm for the Detection of Teeth Occlusions and Gaps

Teeth occlusions and gaps attest to either teeth overlapping along the view line or abnormal space between teeth and sometimes acceptable teeth length mismatch such as with the canine teeth. Vertical integration of Gaussian filters of horizontal gradients along relatively short vertical lines provides a good assessment of such anomalies. An algorithm that performs such depth map gradient requires an inaccurate depth map but a sufficiently sensitive algorithm to capture cliffs in the depth map along the line of view.

As discussed in the Background section above, prior art methods for converting a 2D image to 3D requires knowledge, inter alia, of the intrinsic and extrinsic camera matrices. The presently described method obviates the need for knowledge (e.g., via chessboard calibration etc.) of the intrinsic and extrinsic camera matrices, rather, the instantly disclosed artificial neural network, by default, learns these values and is able to generate a depth map from a 2D image. The presently described U-Net solution can be used for a variety of purposes. One such purpose is to replace the method for generating a depth map by calculating the depth gradients as discussed above. The depth map generated by

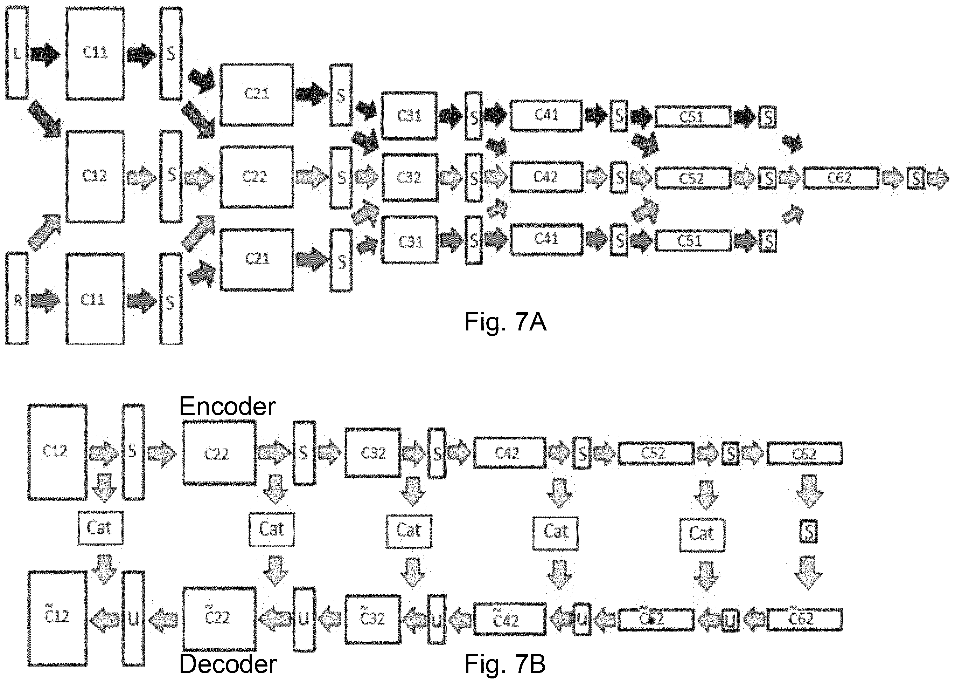

There is described hereafter a stereographic U-Net based solution which is based on supervised learning. It is possible to interpret a middle row of the layers in the encoder as self-supervised (see Fig. 7A).

OVERVIEW

The idea is to provide a U-Net neural network that computes a depth map for a left image from a pair of right and left images.

Next, we train the depth map U-Net and calculate a discrete Dice Loss function. The Dice Loss function was initially designed for segmentation tasks and not for a depth map. The present depth map U-Net is trained to identify depth. So, instead of the U-Net providing semantic segmentation, which is the task of assigning a class label to every single pixel of an input image, the depth map U-Net assigns a depth value (a z-axis value) to every pixel. To that end, the depth training label is separated into 8-32 values from the maximal value to zero. This method is known as the method of bins (binning method) and is widely used in other areas of image processing such as in histogram-based image methods. For each one of the, for e.g., 32, bins the Mean Square Root Error is calculated separately.

$$Error_k = \sqrt{\frac{\sum_{i=0}^{Height-1} \sum_{j=0}^{Width-1} \chi(i,j,k)(Output(i,j) - Label(i,j))^2}{\sum_{i=0}^{Height-1} \sum_{j=0}^{Width-1} \chi(i,j,k)}}$$

$$Error = \sum_{k=0}^{31} Error_k$$

$$\chi(i,j,k) = \begin{cases} 1 & \frac{MaxDepth}{32} k < Label(i,j) \leq \frac{MaxDepth}{32}(k+1) \\ 0 & Otherwise \end{cases}$$

A Depth Map U-Net (Binocular U-Net)

U-Nets are artificial neural networks which consist of a down-sampling encoder and an up-sampling decoder while bridges of information flow exist between the encoder and the decoder.

The instant dedicated model is based on a special design of an autoencoder which is motivated by the structure of the striate visual cortex in which there are areas designated for input from the left eye, the right eye, and both eyes.

Figure 7A depicts a diagram of an example encoder of the depth map U-Net of the invention. The instant encoder differs from a usual U-Net encoder. Figure 7B illustrates a diagram of a regular autoencoder. Comparing the encoders of Fig. 7A and 7B, one can see that only the middle encoder of the instant (depth map) encoder of Fig. 7A is used in the regular autoencoder of Fig. 7B.

Fig. 7B depicts an ordinary autoencoder structure with skip connections that concatenate "Cat" the lower decoder input with activations of the encoder layers. The "U" layers stand for bilinear interpolation up-sampling layers.

In an alternative implementation to the preferred embodiment, the up-sampling can be done with Transposed Convolution layers. In the preferred embodiment c12, c22, c32, c42, c52, c62 are not single convolution layers. Each c layer comprises two convolution layers followed by a Batch Normalization layer. In the decoder, each $\tilde{c}$ layer comprises two convolution layers followed by Batch Normalization.

Optional residual layers

Each C layer of the encoder has the option of an additional residual layer so a convolutional layer with (Input_channels, Output_channels, Width, Hight, kernel(w,h), padding(pw, ph)) which outputs output channels, output_width, output_height is fed into a convolutional layer with kernel(3,3), padding (1,1) and the same number of input and output channels. For example, if the output to layer C is 48 * 160 *120 then an additional residual layer CR will also output 48 * 160 * 120 with input channels 48, input height 160 and input width 120 and output 48 channels, output height 160 and output width 120 and the residual results is then the addition of the output of C and CR.

The following layout of the encoder is simple without additional residual layers. It also does not include additional C61 convolutional layers that are used for additional parameters that the encoder outputs.

**The encoder layers**

Layer 1 receives left image, right image 3 * 640 * 480

Left C11 - Input left 3 * 640 * 480, kernel (3, 3), padding (1,1) output 24 * 640 * 480 S - Max pooling.

Batch norm.

Right C11 - Input left 3 * 640 * 480, kernel (3, 3), padding (1,1) output 24 * 640 * 480 S - Max pooling.

Batch norm.

Middle C12 - Input left and right 6 * 640 * 480, kernel (3, 3), padding (1,1) output 24

* 640 * 480

S - Max pooling.

Batch norm.

Layer 2 receives left input, middle input, right input.

Left C21 - Input left C11 - 24 * 320 * 240, kernel (3, 3), padding (1,1) output 48 * 320 * 240

S - Max pooling.

Batch norm.

Right C21 - Input right C11 - 24 * 320 * 240, kernel (3, 3), padding (1,1) output 48 * 320 * 240

S - Max pooling.

Batch norm.

Middle C22 - 2 * C11, C12 - 72 * 320 * 240, kernel (3, 3), padding (1,1) output 48 * 320 * 240

S - Max pooling.

Batch norm.

Layer 3 receives left input, middle input, right input.

Left C31 - Input left C21 - 48 * 160 * 120, kernel (3, 3), padding (1,1) output 96 * 160 * 120

S - Max pooling.

Batch norm.

Right C31 - Input right C21 - 48 * 160 * 120, kernel (3, 3), padding (1,1) output 96 * 160 * 120

S - Max pooling.

Batch norm.

Middle C32 - Input 2xC21, C22 - 144 * 160 * 120, kernel (3, 3), padding (1,1) output 96 * 160 * 120

S - Max pooling.

Batch norm.

Layer 4 receives left input, middle input, right input.

Left C41 - Input left C31 - 96 * 80 * 60, kernel (3, 3), padding (1,1) output 192*80*60 S - Max pooling.

Batch norm.

Right 41 - Input right C31 - 96 * 80 * 60, kernel (3, 3), padding (1,1) output 192*80*60 S - Max pooling.

Batch norm.

Middle C42 - Input 2xC31, C32 - 288 * 80 * 60, kernel (3, 3), padding (1,1) output 192

* 80 * 60

S - Max pooling.

Batch norm.

Layer 5 receives left input, middle input, right input.

Left C51 - Input left C41 - 192 * 40 * 30, kernel (3, 3), padding (1,1) output 384*40*30 S - Max pooling.

Batch norm.

Right C51 - Input right C41 - 192 * 40 * 30, kernel (3, 3), padding (1,1) output 384 * 40 * 40

S - Max pooling.

Batch norm.

Middle C52 - Input 2xC41, C42 - 576 * 40 * 30, kernel (3, 3), padding (1,1) output 384

* 40 * 30

S - Max pooling.

Batch norm.

Layer 6 receives left input, middle input, right input.

Special layer, not in the drawing,

Left C61 - Input left C51 - 384 * 20 * 15, kernel (5, 6), padding (0,0) output 96*16*10 S - Max pooling.

Batch norm.

Special layer, not in the drawing,

Right C61 - Input right C51 - 384*20*15, kernel (5, 6), padding (0,0) output 96*16*10 S - Max pooling.

Batch norm.

Middle C62 - Input 2xC51, C52 - 1152 * 20 * 15, kernel (5, 6), padding (0,0) output 768 * 16 * 10

S - Max pooling.

Batch norm.

**The decoder layers**

It is important not to confuse between the encoder and the decoder layer. For example, layer c5 in the encoder and in the decoder, are two different layers. The main layers of the decoder are denoted by ~, e.g., ~c52. Main layers are made of two convolutional layers, with the second layer serving as a residual layer.

U6: up-sampling receives input from max pooling after c62 and then S as 768 * 8 * 5.
Output: 768 * 16 * 10
Layer ~c62 is made of c6 and c6A.
c6 receives input from max pooling after c62 as 768 * 16 * 10
Output: 384 * 20 * 15. Kernel size (5, 4), padding (4, 4).
c6A receives input from c6 as 384 * 20 * 15.
Output: 384 * 20 * 15. Kernel size (3,3), padding (1,1).
U5: up-sampling from 384 * 20 * 15 to 384 * 40 * 30
Layer ~c52 is made of c5 and c5A.
Concatenation of 384 * 40 * 30 from C52 with 384 * 40 * 30 from U5 into 768*40*30.
c5 receives 768 * 40 * 30 and outputs 192 * 40 * 30. Kernel size (3,3), padding (1,1).
c5A receives 192 * 40 * 30 and outputs 192 * 40 * 30. Kernel size (3,3), padding (1,1).
U4: up-sampling from 192 * 40 * 30 to 192 * 80 * 60
Layer ~c42 is made of c4 and c4A.
Concatenation of 192 * 80 * 60 from C42 with 192 * 80 * 60 from U4 into 384*80*60.
c4 receives 384 * 80 * 60 and outputs 96 * 80 * 60. Kernel size (3,3), padding (1,1).
c4A receives 96 * 80 * 60 and outputs 96 * 80 * 60. Kernel size (3,3), padding (1,1).
U3: up-sampling from 96 * 80 * 60 to 96 * 160 * 120
Layer ~c32 is made of c3 and c3A.
Concatenation of 96*160*120 from C32 with 96*160*120 from U3 into 192*160*120.
c3 receives 192*160*120 and outputs 48 * 160 * 120. Kernel size (3,3), padding (1,1).
c3A receives 48*160*120 and outputs 48 * 160 * 120. Kernel size (3,3), padding (1,1).
U2: up-sampling from 48 * 160 * 120 to 48 * 320 * 240
Layer ~c22 is made of c2 and c2A.
Concatenation of 48*320*240 from C22 with 48 * 320 * 240 from U2 into 96*320*240.
c2 receives 96 * 320 * 240 and outputs 96 * 320 * 240. Kernel size (3,3), padding (1,1).
c3A receives 96*320*240 and outputs 96 * 320 * 240. Kernel size (3,3), padding (1,1).
U1: up-sampling from 24 * 320 * 240 to 12 * 640 * 480
Concatenation of 24*640*480 from C12 with 12*640*480 from U1 into 36*640*480.

There are two versions of layer ~c12:

Layer ~c12 is made of one layer, c1. A last decoder layer outputs either 1 depth value for the left image or 2 depth values for the right image and for the left image. Our preference is 1 such value.

Version 1 - Depth map calculated for the left image - preferred.

c1 receives 36 * 640 * 480 and outputs a single depth map 1 * 640 * 480

This version is designed for training with real world depth labels such as from LIDAR and with a mask that tells where in (y, x) coordinates depth values are available.

Version 2 - Depth map calculated for the right and the left image.

c1 receives 36 * 640 * 480 and outputs 2 * 640 * 480.

**Depth map for larger transformations between left and right**

The following is a depth map neural network in which the encoder adds more horizontal layers in each level of processing in comparison to the previous encoder. One of the innovative steps is the breaking down of the representation of the transformation between left and right images into smaller transformations. This neural network is a stand-alone neural network which is directly trained with depth labels in a supervised manner. This unique process of using asymmetric Encode-Decoder channels (3 in the Encode and 2 in the Decoder), imitates the human Ocular dominance column (shared input between the eyes). Ocular dominance columns are stripes of neurons in the visual cortex of certain mammals (including humans) that respond preferentially to input from one eye or the other. The columns span multiple cortical layers, and are laid out in a striped pattern across the surface of the striate cortex (V1).

An alternative depth map encoder for larger transformations between left and right images requires more intermediate convolutional layers in each level of processing. In the following illustration, S denotes down-sampling by max pooling as before and C denotes two layers followed by batch normalization. Layers that have the same weights have the same name.

Following is an illustration of an alternative encoder based convolutional neural network which is designed to be trained with real world depth, e.g., from LIDAR. The encoder breaks down features which represent transformations between left and right RGB images. There are many ways to define the number of output features of each convolutional layer in this diagram. Preferably the number of features grows with depth and the dimension of the image is reduced. The dimensions after the first 6 levels of processing, before c75, are the same as before. (640, 480) -> (320, 240) -> (160, 120) -> (80, 60)->(40,30)->(20,15)->(8,5). Preferably, c75 outputs 768 up to 1024 channels/features with dimension (8,5).

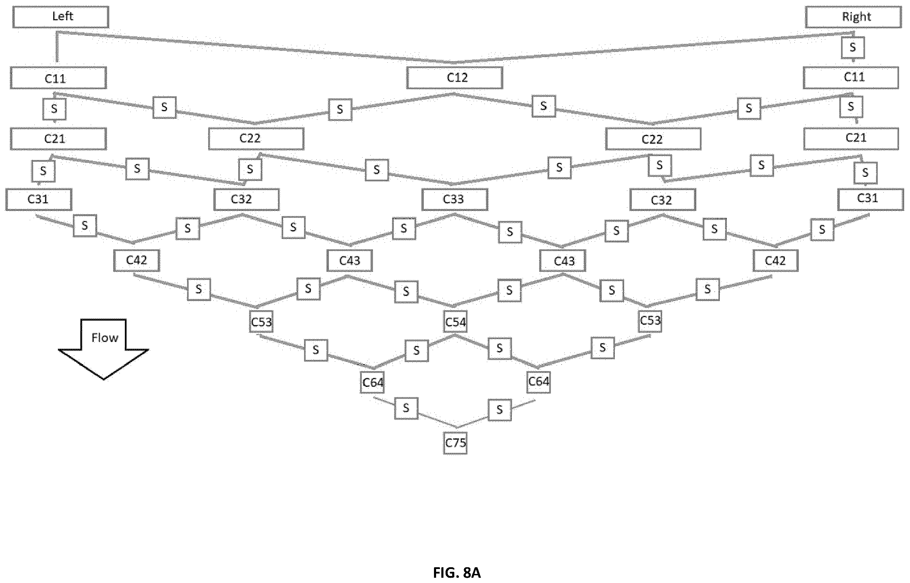

Figure 8A depicts a depth map encoder when the transformation between the left and right image is large. The decoder is simpler and is trained to output the depth map of the left image. Following is an illustration with typical U-Net skip-connections between the left side of the encoder and the decoder on the left. The U layers are up-sampling layers which use the bilinear interpolation to double the dimensions of input. The flow in the decoder is upwards.

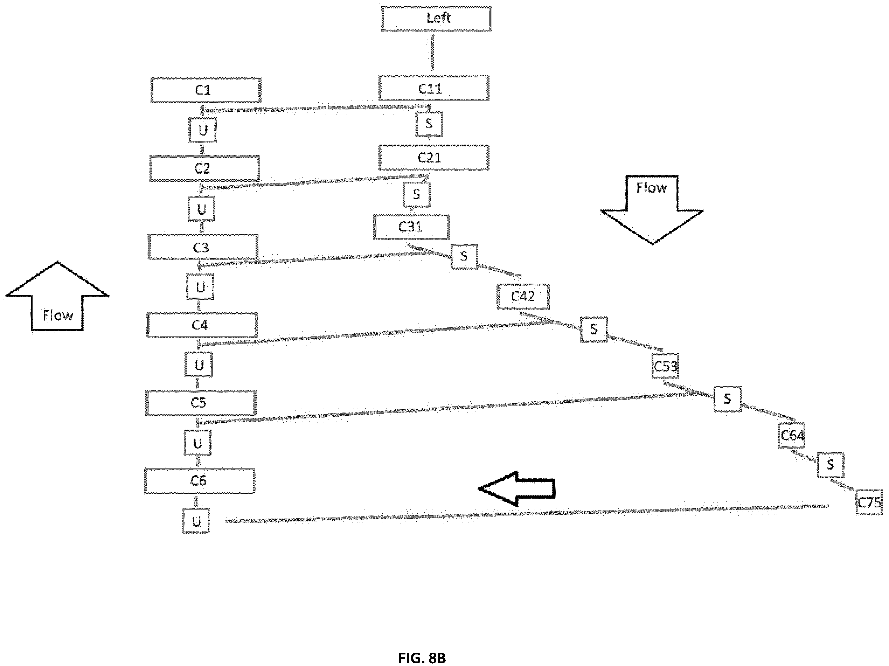

Figure 8B depicts a depth map decoder when the transformation between the left and right image is large. The decoder directly outputs the depth map for the left image. Skip connections concatenate feature maps from the encoder with inputs from preceding layers of the decoder.

Once the Go/No-Go algorithm has determined that the person can be treated with the present system, it is necessary to create an aligner suited specifically to the person. Dental arch alignment is usually done by using dental frets (aligners). There is described hereafter an innovative process that constructs a virtual teeth aligner for each individual patient based on the patient's scanned teeth (upper jaw - Maxilla, or lower - Mandible) using STL 3D teeth structure file. A designated teeth scanner is used which outputs an STL file. The STL file is used to generate surface normal vectors. STL is a file format native to the stereolithography CAD (Computer-Aided Design) software created by 3D Systems, Inc.

The described innovative digitized method includes Machine Learning and special 3D structures processing using STL, Points Cloud and STEP files. The instantly described aligner includes a special balloon that is placed in the aligner with the required space to allow the balloon to be inflated with pressured air pulses and push the patient's teeth to their final aligned position and state. The special teeth aligner performs teeth alignment by using a mold which is fitted to the teeth in which pulsed pneumatic pressure pushes the teeth from within the mouth cavity while the external sides of the teeth are supported by the mold. The purpose of the device is to align the teeth into a continuous smooth dental arch.

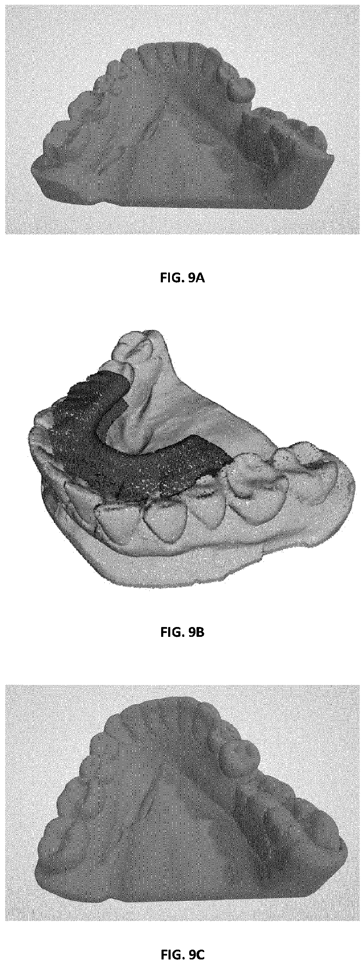

In general, the orthodontal treatment plan is based on a diagnosis test and then based on the patient's teeth scan (STL 3D structure file). Figure 9A depicts a scan of a patient's teeth in STL file format. The aligner design is based on the final position of the aligned teeth and the present teeth structure (upper and/or lower jaws) including the balloon components inside structure. The overall design is based on alignment prediction, e.g., "iOrthoPredictor" by Yang L. et al. which performs silhouette segmentation and is based on a Multilayered Perceptron (MLP) to compute the alignment and is based on a predictive encoder-decoder neural network to predict the final look of the teeth after alignment. A good example of the function of the MLP is to compute 3 points of pressure on each tooth and the direction of the force vectors in each one of these points. Training the MLP requires labels that can be achieved either from professional orthodontists or from a simulator.

Once the final position on the aligned teeth is computed (final patient's teeth state - Positive STL) based on the scanned teeth (STL file of the patient's teeth current state before the alignment treatment - starting state) the next step is to generate the special aligner that fits onto the patient's teeth and includes the special inflating balloon structure. This process includes transforming the Final state STL 3D structure into a points cloud with the required degree of accuracy and resolution. Each positive STL can be translated into 10,000 to 1,000,000 points in a three dimensional space (x,y,z). Figure 9B is an example of a points cloud of the dental depicted in Fig. 9A. The example points cloud is depicted together with a points cloud of a structure for holding the special inflating balloon.

The points cloud is then inflated / enlarged in the positive direction (NORMAL vector) with the required space constructing the aligner that is fitted to the individual patient. Then the new points cloud is translated back to an STL file representing the patient's aligner. Figure 9C depicts an STL file representing the points cloud which was enlarged. (The example STL depicted in Fig. 9C is not actually a final state STL, but rather an enlarged representation of the starting state; the figure depicted is merely for the sake of elucidation of the concept of converting a Positive STL into an enlarged or Negative STL).

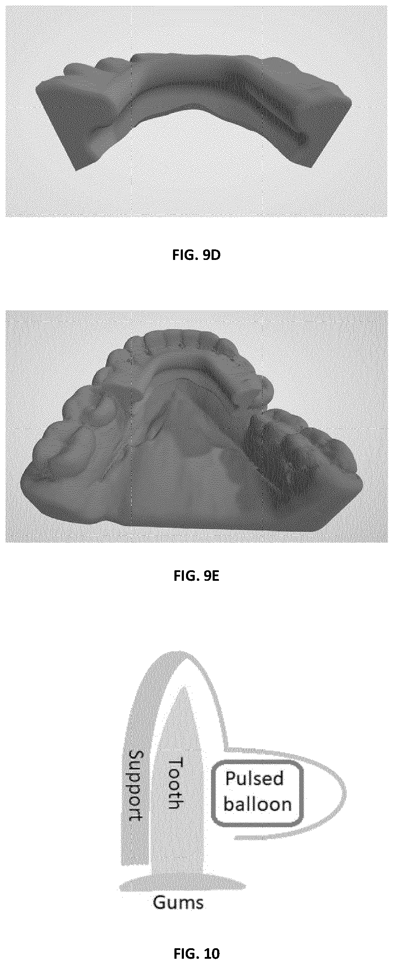

The balloon structure is added with the appropriate space that enables the balloon to push the teeth into their final state. Figure 9D depicts the balloon structure in STL format. This STL is digitally adjusted with clear-cut processing to ensure the patient can take it off and put it on while the special aligner holds tightly to the teeth without loosening. The clear-cut process analysis is based on identifying the 3D structure of each tooth using a ML trained model. The final cut is done along the external side of each tooth to enable taking the aligner off with ease. After this clear-cut processing, the processed STL can be converted into STEP non-parametric file using NURBS (Non-Uniform Rational B-Splines Modeling) processing and then it can be printed using a special 3D printer using the right materials such as silicon and plastic layers.

The process described above can be summarized as follows: After a decision that teeth alignment is feasible, the teeth are scanned with a designated teeth scanner which outputs an STL file. The STL file is used to generate surface normal vectors. The 3D scanner achieves the data acquisition and presents the 3D scan as a mesh.

The normal vectors to the mesh (vectors normal to virtual planes of small groups of points) are calculated and an inflated mesh is calculated by adding, for e.g., 1mm to 2mm gap along the normal vectors. Fig. 9A depicts an image of the teeth surfaces without inflation. Fig. 9C depicts an image of the teeth surfaces after inflation. The inflated mold (see Fig. 9C) is aligned with the structure (see Fig. 9D) that provides forces in order to gradually move the teeth by pulsed force. Figure 9E depicts a composite mold of the inflated teeth and aligned structure.

The matching between the structure and the teeth, in one example embodiment, is based on the following steps:

1) Find the two largest PCA eigenvectors of the points of the teeth in relation to its center of gravity.
2) Find the two largest PCA eigenvectors of the points of the structure in relation to its center of gravity.
3) Align the two centers of gravity and rotate the structure such that the eigenvectors with the largest eigenvalues are aligned.
4) Randomly select a small sub sample of points on the inner side of the front teeth.
5) Find external points on the structure that best match the randomly selected teeth points.
6) Use the Hungarian matching algorithm to find the best match (linear_sum_assignment in scipy.optimize in Python).
7) From the best match between the random sampled points of the teeth and the structure and from the current positions of the structure and the teeth, calculate a small rotation and a small translation that reduces the overall distance between the points on the teeth and the points of the structure.
8) Update the position of the center of gravity of the structure and the rotation matrix of the structure in relation to the teeth.
9) Loop to (4) for several tens of iterations, 128 in preferred embodiment. Possibly (9) can stop if the error sum is below a certain value.

A mold that wraps the teeth is drawn for a production phase. Matching an ideal dental arch is done by software. First, a teeth segmentation U-Net is responsible for determining the segmentation of the teeth from more than one 2D projection of the dental points cloud. The points cloud is turned into an STL. This STL is 3D printed into a physical aligner. The displacement in relation to the ideal dental arch is calculated by software, e.g., "iOrthoPredictor" along with the placement of the 3D points in relation to the ideal dental arch. The delta, between the ideal location of each point and the actual points of a tooth that must be moved, attaches a flow vector to each point on the tooth surface. The force vectors that must be applied are proportional to the negative sign of the displacements. In fact, it is sufficient to calculate the force 1 mm above the center of the tooth from behind the tooth and in left and right bottom points 1mm above the gums from behind the tooth. These points are selected by the sign of their normal vector. Points behind the teeth have a normal vector pointing inward the mouth cavity. The average of all the force lines is the translational force.

$$\bar{F} = -\alpha \frac{1}{n} \sum_{k=0}^{n-1} \bar{t_k} \qquad (1)$$

Where $\alpha$ is a constant that depends on the hardware and is controllable, $\bar{t}$ is a three-dimensional translation vector $\bar{t_k} = (x_k - X_k, y_k - Y_k, z_k - Z_k)$ where capital letters represent the ideal location and small letters represent the actual position. $n$ is the number of 3D points of the chosen tooth. The simpler form of the invention is of a balloon that is pulsed with compressed air within as mold as depicted in Fig. XX-C. The more advanced invention has 3 balloons in which a pulsed air pressure exerts force on the tooth and the advanced invention generates a torque that is delivered to the tooth.

Next, the torque $\bar{T}$ is calculated with the cross product,

$$\bar{T} = -\alpha \frac{1}{n} \sum_{k=0}^{n-1} \bar{r_k} \times \bar{t_k} \qquad (2)$$

$$\bar{r_k} = (x_k - x, y_k - y, z_k - z)$$

$$\bar{c} = (x, y, z) = \frac{1}{n} \sum_{k=0}^{n-1} (x_k, y_k, z_k)$$

So, choosing 3 points $p_1$, $p_2$, $p_3$ where the force has to be applied, we have the following vector equations.

$$\bar{F}_1 + \bar{F}_2 + \bar{F}_3 = \bar{F} = -\alpha \frac{1}{n} \sum_{k=0}^{n-1} \bar{t_k} \qquad (3)$$

$$\bar{c} = (x, y, z)$$

$$\text{Let } \bar{q}_1 = (\overline{p_1} - \bar{c}), \bar{q}_2 = (\overline{p_2} - \bar{c}), \bar{q}_3 = (\overline{p_3} - \bar{c}) \qquad (4)$$

$$\bar{F}_1 \times \bar{q}_1 + \overline{F_2} \times \bar{q}_2 + \bar{F}_3 \times \bar{q}_3 = \bar{T} = -\alpha \frac{1}{n} \sum_{k=0}^{n-1} \bar{r_k} \times \bar{t_k}$$

Equations (3) and (4) do not have only one solution and therefore another condition is imposed on the 3 force vectors which consist altogether of 9 components.

To this can be added one more equation, such as equal norms $\|\bar{F}_1\| = \|\bar{F}_2\| = \|\bar{F}_3\|$.

The mold is produced with pressure-pulsed balloons for teeth that must be moved in order to align the dental arch. Figure 10 is a diagrammatic representation of a cross-sectional view of an aligner seated over a tooth. The lower part of the mold is cut out in order that due to the convexity of the teeth, the mold will not become stuck on convex teeth.

In the one balloon per tooth version of the invention, the balloons are adjusted to generate a pulsing force in the direction of $\bar{F}$, see (3). In the 3 balloons per tooth version of the invention each one of three balloons are aligned along $\bar{F}_1$, $\bar{F}_2$ and $\bar{F}_3$ and the forces are generated by the same pulsed source and therefore $\|\bar{F}_1\| = \|\bar{F}_2\| = \|\bar{F}_3\|$. The maximal forces are the multiplication of the contact area with a tooth multiplied by the maximal pressure amplitude of the pulsed air pressure.

A summary of the system and process including some additional components and steps is detailed below: The system includes, inter-alia, a computer, memory and processor. A non-transitory computer-readable medium comprises instructions stored thereon, that when executed on the processor perform one or more of the tasks detailed hereafter. Additional details of the systems are detailed below.

1) Receiving a 3-dimensional (3D) scan of a dental arch.

2) Analyzing the scanned patient's teeth using STL 3D file to get a manifold of teeth representing the patient's final aligned teeth position in a 3D space. A manifold, as understood herein, is a collection of points forming a certain kind of set, such as those of a topologically closed surface or an analog of this in three or more dimensions.

3) Converting the final state STL into a points cloud.

4) Creation of a mold by expanding the teeth manifold along the surface normal vectors, i.e., adding 1mm to 2mm gap along each outwards bound surface normal vector.

5) Computing pressure points and force vectors on the virtual aligner.

6) Fitting pneumatic cushions in the internal side of the mold.

7) Combining a points cloud of a balloon structure to the points cloud of the manifold.

8) Converting the aligner points cloud into a representation of an aligner in a 3D printable file format.

9) Performing a clear-cut process based on identifying each covered tooth by the aligner enabling taking the aligner off and putting it on with ease while the aligner is held tightly on the teeth without loosening.

10) Applying a pulsed force through the pneumatic cushions.

11) Simulating the teeth motion in different days of treatment and adjusting the force vectors in order to achieve the best alignment result.

12) Printing an aligner on a 3D printer from the representation of the aligner in the 3D printable file format.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, non-transitory storage media such as a magnetic hard-disk and/or removable media, for storing instructions and/or data. Optionally, a network connection is provided as well. A display and/or a user input device such as a keyboard or mouse are optionally provided as well.

For example, any combination of one or more non-transitory computer readable (storage) medium(s) may be utilized in accordance with the above-listed embodiments of the present invention. A non-transitory computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: a portable computer diskette, a hard disk, a random-access memory (RAM), a read-only memory (ROM), an erasable programmable read-only memory (EPROM or Flash memory), a portable compact disc read-only memory (CD-ROM), an optical storage device, a magnetic storage device, or any suitable combination of the foregoing. In the context of this document, a computer readable non-transitory storage medium may be any tangible medium that can contain or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

As will be understood with reference to the paragraphs and the referenced drawings, provided above, various embodiments of computer-implemented methods are provided herein, some of which can be performed by various embodiments of apparatuses and systems described herein and some of which can be performed according to instructions stored in non-transitory computer-readable storage media described herein. Still, some embodiments of computer-implemented methods provided herein can be performed by other apparatuses or systems and can be performed according to instructions stored in computer-readable storage media other than that described herein, as will become apparent to those having skill in the art with reference to the embodiments described herein. Any reference to systems and computer-readable storage media with respect to the following computer-implemented methods is provided for explanatory purposes and is not intended to limit any of such systems and any of such non-transitory computer-readable storage media with regard to embodiments of computer-implemented methods described above. Likewise, any reference to the following computer-implemented methods with respect to systems and computer-readable storage media is provided for explanatory purposes and is not intended to limit any of such computer-implemented methods disclosed herein.

The flowcharts and block diagrams in the Figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The descriptions of the various embodiments of the present invention have been presented for purposes of illustration but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

As used herein, the singular form "a", "an" and "the" include plural references unless the context clearly dictates otherwise.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

The above-described processes including portions thereof can be performed by software, hardware and combinations thereof. These processes and portions thereof can be performed by computers, computer-type devices, workstations, processors, microprocessors, other electronic searching tools and memory and other non-transitory storage-type devices associated therewith. The processes and portions thereof can also be embodied in programmable non-transitory storage media, for example, compact discs (CDs) or other discs including magnetic, optical, etc., readable by a machine or the like, or other computer usable storage media, including magnetic, optical, or semiconductor storage, or other source of electronic signals.

The processes (methods) and systems, including components thereof, herein have been described with exemplary reference to specific hardware and software. The processes (methods) have been described as exemplary, whereby specific steps and their order can be omitted and/or changed by persons of ordinary skill in the art to reduce these embodiments to practice without undue experimentation. The processes (methods) and systems have been described in a manner sufficient to enable persons of ordinary skill in the art to readily adapt other hardware and software as may be needed to reduce any of the embodiments to practice without undue experimentation and using conventional techniques.

While the invention has been described with respect to a limited number of embodiments, it will be appreciated that many variations, modifications and other applications of the invention may be made. Therefore, the claimed invention as recited in the claims that follow is not limited to the embodiments described herein.

**Claims**

**1.** A method comprising:

obtaining a plurality of images in which front teeth of a subject are visible;
performing segmentation on selected images from the plurality of images to create a first segmentation mask and labeling each tooth in the selected images to provide a detailed segmentation map;
generating a depth map of the front teeth;
calculating a horizontal gradient of the depth map and a vertical moving average of a plurality of pixels of the horizontal gradient to receive depth gradients and flagging depth gradients where the vertical moving average exceeds a predefined threshold or is classified by an Artificial Neural Network as abnormal;
inputting the depth gradients and the detailed segmentation map into a classifier to determine whether the front teeth are within predetermined parameters; and

receiving a go or no-go classification from the classifier.

2. The method of claim 1, wherein performing segmentation includes a preprocessing step of denoising and features space analysis adapted to segment teeth from other elements in each of the selected images.

3. The method of claim 2, wherein the detailed segmentation map is generated by employing an artificial neural network (ANN) to recognize each tooth and label each tooth with clear edges thereof.

4. The method of claim 1, wherein the depth map is generated by a specialized U-Net trained on a dataset of images of teeth in different configurations.

5. The method of claim 1, wherein the classifier is a convolution neural network.

6. A method for generating a depth map from a 2-dimensional image, comprising:

training a depth map U-Net neural network on a dataset of images, wherein a depth value of each pixel in each image is known;

inputting the 2-dimensional image to the depth map U-Net; and

outputting, by the depth map U-Net, the depth map of the 2-dimensional image.

7. The method of claim 6, wherein the depth map U-Net has an asymmetric three-channel encoder and a two-channel decoder.

8. The method of claim 7, wherein the three-channel encoder has a left propagation path, a right propagation path and a middle propagation path.

9. The method of claim 8, wherein the middle propagation path is self-supervised.

10. A non-transitory computer-readable medium comprises instructions stored thereon, that when executed on a processor perform a method of generating a final state teeth aligner structure file, comprising:

receiving a 3-dimensional (3D) scan of a dental arch;

analyzing the 3D scan to get a manifold of teeth representing a final aligned teeth position in a 3D space;

converting the manifold of teeth into a points cloud;

generating a representation of a mold by expanding the manifold of teeth along surface normal vectors thereof;

combining a points cloud of a balloon structure to the points cloud of the manifold to receive an aligner points cloud;

converting the aligner points cloud into a representation of an aligner in a 3D printable file format.

11. The method of claim 10, further comprising: printing an aligner on a 3D printer from the representation of the aligner in the 3D printable file format.

**FIG. 1**

100

Acquire set of 2D images
Filter for noise – 102.1
Extract mouth rectangle – 102.2

102

Perform segmentation on 2D images.

104

Preprocess images to segment teeth from other.

104.1

Use segmentation to label each tooth.

104.2

Generate a points cloud depth map of the front teeth

106

Scanning and smoothing depth map to receive depth gradients and flag abnormal gradients

108

Input depth gradients into a classifier

110

Receive a go or no-go classification from the classifier

112

FIG. 2a                    FIG. 2b                    FIG. 2c

FIG. 3a

FIG. 3b

**FIG. 3c**

FIG. 3d

FIG. 3e

**FIG. 4a**

**FIG. 4b**

**FIG. 4c**

FIG. 5a

FIG. 5b

FIG. 5c

**PRIOR ART**

**Fig. 6**

Fig. 7A

Fig. 7B

Encoder

Decoder

FIG. 8A

EP 4 372 681 A2

FIG. 8B

FIG. 9A

FIG. 9B

FIG. 9C

**FIG. 9D**

**FIG. 9E**

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63426078
- US 63426084
- US 63426088

**Non-patent literature cited in the description**

- **YANG L.** *iOrthoPredictor*